# EUROPEAN PATENT APPLICATION

(11) **EP 4 400 481 A1**
(43) Date of publication of application: **17.07.2024**
(21) Application number: 22864175.9
(22) Date of filing: 04.08.2022
(51) Int. Cl.: C02F 1/461, C25B 9/00, C25B 15/023, C25B 15/029, C25B 15/08, C25B 1/13

(54) **ELECTROLYTIC LIQUID GENERATION SYSTEM AND CONTROL SYSTEM**

(30) Priority: 06.09.2021 JP 2021144954
(71) Applicant: Panasonic Intellectual Property Management Co., Ltd., Osaka-shi, Osaka 540-6207 (JP)
(72) Inventor: HOTTA, Mami, Kadoma-shi Osaka 571-0057 (JP); II, Chihiro, Kadoma-shi Osaka 571-0057 (JP); INAGAKI, Kenichiro, Kadoma-shi Osaka 571-0057 (JP)
(74) Representative: Grünecker Patent- und Rechtsanwälte PartG mbB
(86) International application number: PCT/JP2022/029998
(87) International publication number: WO 2023/032589

(57) **Abstract**

Electrolytic liquid generation system (1) includes functional unit (4) that comprises an electrolytic liquid generation function of electrolyzing tap water (W1) fed from liquid inflow path (31) to generate ozone water (W2) from tap water (W1), and a disinfection function of disinfecting tap water (W1). Controller (5) controls at least one of the electrolytic liquid generation function or the disinfection function of functional unit (4). Controller (5) improves the electrolytic liquid generation function in the first control mode more than in the second control mode. Controller (5) improves the disinfection function in the second control mode more than in the first control mode.

## Description

### TECHNICAL FIELD

The present disclosure relates to an electrolytic liquid generation system and a control system.

### BACKGROUND ART

Conventionally known examples of an electrolytic liquid generation system include an ozone water generator that generates ozone water (electrolytic liquid) in which ozone (electrolytic product) is dissolved in water. An electrolytic part includes a conductive film provided between an anode and a cathode. Electrolytic treatment is performed by generating a potential difference between the anode and the cathode in a state where the electrolytic part is immersed in water. The electrolytic treatment causes an electrochemical reaction in water to generate ozone water (e.g., see PTL 1). The generated ozone water is then flown to an object such as a pipeline, a toilet, a washbasin, a bathroom, or a kitchen, thereby exerting an effect such as disinfection, mold prevention, or deodorization on the object.

### Citation List

### Patent Literature

PTL 1: Unexamined Japanese Patent Publication No. 2017-176993

### SUMMARY OF THE INVENTION

The conventional electrolytic liquid generation system exerts an effect relevant to disinfection of a liquid to be treated (referred to below simply as "liquid"). Unfortunately, the conventional electrolytic liquid generation system has difficulty in adjusting a balance between disinfection of a liquid and generation efficiency of an electrolytic liquid.

It is an object of the present disclosure to provide an electrolytic liquid generation system and a control system that are capable of adjusting a balance between disinfection of a liquid and generation efficiency of an electrolytic liquid.

An electrolytic liquid generation system according to an aspect of the present disclosure includes a liquid flow path, a functional unit, and a controller. The liquid flow path allows a liquid to flow therethrough. The functional unit has an electrolytic liquid generation function of generating an electrolytic liquid from the liquid by electrolyzing the liquid fed through the liquid flow path, and a disinfection function of disinfecting the liquid. The controller controls at least one of the electrolytic liquid generation function or the disinfection function of the functional unit. The controller is configured to operate in any one of multiple control modes including at least a first control mode and a second control mode. The controller is configured to improve the electrolytic liquid generation function in the first control mode more than in the second control mode. The controller is configured to improve the disinfection function in the second control mode more than in the first control mode.

A control system according to another aspect of the present disclosure is used for an electrolytic liquid generation system having an electrolytic liquid generation function of generating an electrolytic liquid from a liquid by electrolyzing the liquid and a disinfection function of disinfecting the liquid. The control system includes a controller that controls at least one of the electrolytic liquid generation function or the disinfection function. The controller is configured to operate in any one of multiple control modes including at least a first control mode and a second control mode. The controller is configured to improve the electrolytic liquid generation function in the first control mode more than in the second control mode. The controller is configured to improve the disinfection function in the second control mode more than in the first control mode.

The present disclosure exerts an effect of being capable of adjusting a balance between disinfection of a liquid and generation efficiency of an electrolytic liquid.

### BRIEF DESCRIPTION OF THE DRAWINGS

Fig. 1 is a block diagram illustrating an electrolytic liquid generation system according to a first exemplary embodiment.
Fig. 2 is an exploded perspective view illustrating an electrolytic part provided in the electrolytic liquid generation system.
Fig. 3 is a block diagram illustrating a first modification of the electrolytic liquid generation system.
Fig. 4 is a block diagram illustrating a second modification of the electrolytic liquid generation system.
Fig. 5 is a block diagram illustrating a third modification of the electrolytic liquid generation system.
Fig. 6 is an external view illustrating an example of a water purifier according to a second exemplary embodiment.
Fig. 7 is an external view illustrating an example of a mist cooler of a third exemplary embodiment.

### DESCRIPTION OF EMBODIMENT

Exemplary embodiments each described herein relate generally to an electrolytic liquid generation system and a control system. More specifically, the exemplary embodiments each relate to the electrolytic liquid generation system that generates an electrolytic liquid by electrolyzing a liquid to be treated, and the control system.

Hereinafter, the electrolytic liquid generation system and the control system according to each exemplary embodiment will be described in detail with reference to the drawings. However, the drawings described in the exemplary embodiments below are each a schematic diagram in which ratios of size and thickness of each component do not always reflect actual dimensional ratios.

The exemplary embodiments described below are each merely an example of exemplary embodiments of the present disclosure. The present disclosure is not limited to the exemplary embodiments below, and various modifications can be made depending on design and the like as long as the effects of the present disclosure can be achieved.

### (1) First exemplary embodiment

### (Overview)

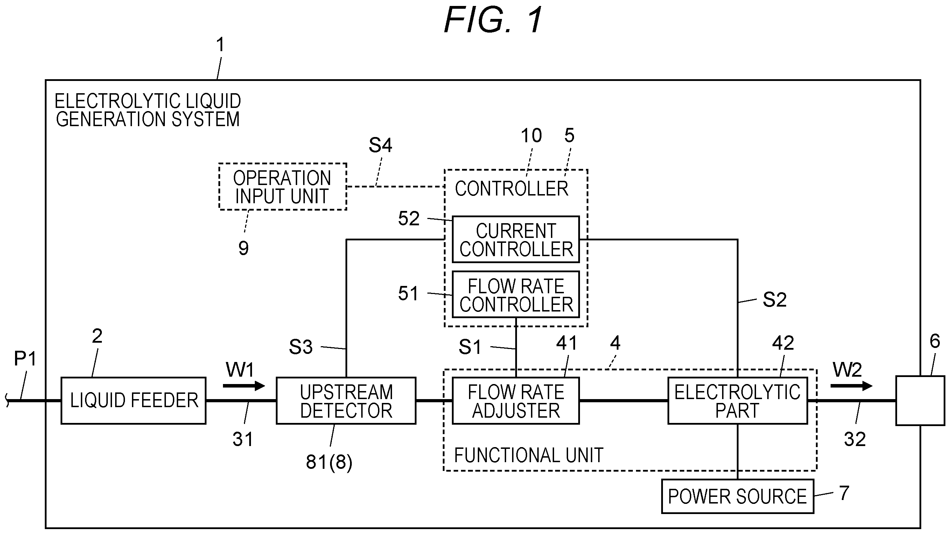

First, an overview of electrolytic liquid generation system 1 according to the present exemplary embodiment will be described with reference to Fig. 1. Fig. 1 is a block diagram illustrating electrolytic liquid generation system 1 according to a first exemplary embodiment.

Electrolytic liquid generation system 1 generates an electrolytic liquid from a liquid by electrolyzing the liquid. Electrolytic liquid generation system 1 of the present exemplary embodiment is an ozone water generator that uses tap water W1 fed from waterworks P1 as a liquid and generates ozone water W2 as an electrolytic liquid. Electrolytic liquid generation system 1 serving as the ozone water generator generates ozone (electrolytic product) by electrolytic treatment for electrolyzing tap water W1 which is a liquid, and dissolves the ozone in tap water W1 to generate ozone water W2 which is an electrolytic liquid. Ozone water W2 is effective for disinfection, deodorization, and organic substance decomposition, for example, and thus is widely used in various fields such as a water treatment field, a hygiene field, a food field, and a medical field.

Electrolytic liquid generation system 1 includes liquid inflow path 31, functional unit 4, and controller 5. Liquid inflow path 31 allows tap water W1 to flow therethrough as a liquid, for example. Functional unit 4 electrolyzes tap water W1 fed from liquid inflow path 31 to perform a disinfection function of disinfecting tap water W1 while performing an electrolytic liquid generation function of generating ozone water W2 as an electrolytic liquid from tap water W1. Controller 5 controls at least one of the electrolytic liquid generation function or the disinfection function of functional unit 4. Controller 5 operates in any one of multiple control modes including at least a first control mode and a second control mode. Controller 5 improves the electrolytic liquid generation function in the first control mode more than in the second control mode. Controller 5 improves the disinfection function in the second control mode more than in the first control mode.

Electrolytic liquid generation system 1 operates in any of the multiple control modes including the first control mode and the second control mode that are different in balance (priority) between the electrolytic liquid generation function and the disinfection function from each other. As a result, electrolytic liquid generation system 1 can adjust balance between disinfection of tap water W1 and generation efficiency of ozone water W2. That is, electrolytic liquid generation system 1 can adjust the balance between the disinfection function and the electrolytic liquid generation function as necessary. For example, electrolytic liquid generation system 1 can adjust the disinfection function and the electrolytic liquid generation function so that the disinfection function can be favorably utilized as necessary.

Electrolytic liquid generation system 1 as described above can be used for a device using an electrolytic liquid such as ozone water W2, such as a water purifier, a mist generator, a washing machine, a dishwasher, a warm-water cleaning toilet seat, a refrigerator, a hot and cold water supply device, a disinfection device, a medical device, an air conditioner, or a kitchen device.

Controller 5 described above constitutes control system 10, and control system 10 can also achieve the above effect of electrolytic liquid generation system 1.

### (Details)

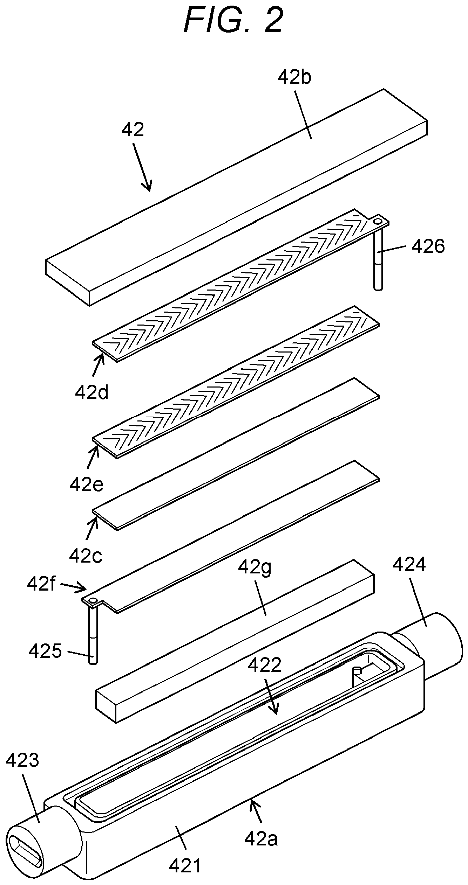

Next, details of electrolytic liquid generation system 1 according to the present exemplary embodiment will be described with reference to Figs. 1 and 2. Fig. 2 is an exploded perspective view illustrating electrolytic part 42 provided in electrolytic liquid generation system 1.

### (1.1) Overall configuration

As illustrated in Fig. 1, electrolytic liquid generation system 1 according to the present exemplary embodiment is an electrolytic liquid generator including liquid feeder 2, liquid inflow path 31, liquid outflow path 32, functional unit 4, controller 5, water feed port 6, power source 7, and detector 8. The electrolytic liquid generator has a configuration in which liquid feeder 2, liquid inflow path 31, liquid outflow path 32, functional unit 4, controller 5, water feed port 6, power source 7, and detector 8 are provided in one housing.

### (1.1.1) Liquid feeder

Liquid feeder 2 includes a feedwater pump to pressurize tap water W1 fed from waterworks P1, and discharge pressurized tap water W1 to liquid inflow path 31. Liquid feeder 2 may further include a tank for storing tap water W1.

Liquid feeder 2 does not need to include a feedwater pump and a tank, and may discharge tap water W1 directly received from waterworks P1 to liquid inflow path 31.

### (1.1.2) Liquid inflow path

Liquid inflow path 31 corresponds to the liquid flow path of the present disclosure, and is a water pipe such as a hose, a resin pipe, or a metal pipe. Liquid inflow path 31 has an inflow port connected to liquid feeder 2 and receives tap water W1 from liquid feeder 2 through the inflow port. Liquid inflow path 31 has an outflow port connected to functional unit 4, and tap water W1 flowing through liquid inflow path 31 is fed to functional unit 4 through the outflow port.

### (1.1.3) Functional unit

Functional unit 4 includes flow rate adjuster 41 and electrolytic part 42. Functional unit 4 including flow rate adjuster 41 and electrolytic part 42 electrolyzes tap water W1 fed from liquid inflow path 31 to perform a disinfection function of disinfecting tap water W1 while performing an electrolytic liquid generation function of generating ozone water W2 from tap water W1. The disinfection function is to generate ozone water W2 having less bacteria by applying electrolytic treatment to tap water W1 to decrease (disinfect) bacteria contained in tap water W1.

### (Flow rate adjuster)

Flow rate adjuster 41 is connected between the outflow port of liquid inflow path 31 and electrolytic part 42. Flow rate adjuster 41 includes an electromagnetic valve and the like, and adjusts a flow rate of tap water W1 fed from liquid inflow path 31 to electrolytic part 42 by adjusting an opening degree of the electromagnetic valve. Hereinafter, the flow rate of tap water W1 fed from liquid inflow path 31 to electrolytic part 42 may be referred to as a liquid feed rate.

Flow rate adjuster 41 is notified of an instruction value (flow rate instruction value) of the liquid feed rate by flow rate instruction signal S1 from controller 5, and adjusts an opening degree of the electromagnetic valve so that the liquid feed rate equals the flow rate instruction value.

Thus, functional unit 4 can adjust the amount of ozone water W2 generated by electrolytic part 42 by adjusting the liquid feed rate using flow rate adjuster 41. That is, functional unit 4 can increase the amount of generation of ozone water W2 by increasing the liquid feed rate using flow rate adjuster 41, and as a result, the electrolytic liquid generation function can be improved. Alternatively, functional unit 4 can decrease the amount of generation of ozone water W2 by decreasing the liquid feed rate using flow rate adjuster 41, and as a result, the electrolytic liquid generation function can be deteriorated.

### (Electrolytic part)

Electrolytic part 42 generates ozone by electrolyzing tap water W1 at a flow rate adjusted by flow rate adjuster 41, and dissolves the ozone in tap water W1 to generate ozone water W2 as an electrolytic liquid.

As illustrated in Fig. 2, electrolytic part 42 includes case 42a, cover 42b, anode (electrode) 42c, cathode (electrode) 42d, conductive film 42e, power feeder 42f, and elastic body 42g.

Case 42a and cover 42b are each made of a non-conductive resin such as acrylic. Case 42a includes case body 421 in a hollow rectangular-parallelepiped shape. Case body 421 includes recess 422 on the one surface thereof. Anode 42c, cathode 42d, conductive film 42e, power feeder 42f, and elastic body 42g are housed in recess 422. Cover 42b serves to cover an opening of recess 422. That is, case 42a and cover 42b constitute a housing in a hollow rectangular-parallelepiped shape.

Upstream connector 423 in a tubular shape is formed at the first end of case body 421 in a longitudinal direction, and downstream connector 424 in a tubular shape is formed at the second end of case body 421 in the longitudinal direction. Upstream connector 423 is connected to a pipe through which tap water W1 at a flow rate adjusted by flow rate adjuster 41 (see Fig. 1) flows, and guides tap water W1, which has passed through flow rate adjuster 41, to the inside of recess 422 of case body 421. Downstream connector 424 is connected to the inflow port of liquid outflow path 32 (see Fig. 1), and guides ozone water W2, which has been generated by electrolytic part 42, from recess 422 to liquid outflow path 32.

Recess 422 houses elastic body 42g, power feeder 42f, anode 42c, conductive film 42e, and cathode 42d. Elastic body 42g, power feeder 42f, anode 42c, conductive film 42e, and cathode 42d are disposed and stacked on a bottom surface of recess 422, and elastic body 42g, power feeder 42f, anode 42c, conductive film 42e, and cathode 42d are stacked in this order from the bottom surface of recess 422.

Elastic body 42g is formed in a rectangular-parallelepiped shape using a material having elasticity such as rubber, plastic, or a metal spring. Power feeder 42f is formed in a rectangular plate shape using titanium, for example. Anode 42c is formed by forming a conductive diamond film on a conductive substrate in a rectangular plate shape using silicon, for example. Conductive film 42e is an ion exchange film of a proton conduction type having a thickness of about 100 µm to 200 µm, for example, and is formed in a rectangular plate shape. Cathode 42d is formed in a rectangular plate shape having a thickness of about 0.5 mm using a stainless alloy, for example.

Elastic body 42g has a first surface in contact with the bottom surface of recess 422. Power feeder 42f has a first surface in contact with a second surface of elastic body 42g, and has a second surface in contact with a first surface of anode 42c. Anode 42c has a second surface in contact with a first surface of conductive film 42e. Conductive film 42e has a second surface in contact with a first surface of cathode 42d. Cathode 42d has a second surface facing a back surface of cover 42b.

Power feeder 42f has one end in the longitudinal direction to which anode power-feeding shaft 425 in a rod shape is attached. Anode power-feeding shaft 425 is connected to power source 7 (see Fig. 1) by wiring. Power feeder 42f is in contact with anode 42c. Anode power-feeding shaft 425 is electrically connected to anode 42c. Cathode 42d has one end in the longitudinal direction to which cathode power-feeding shaft 426 in a rod shape is attached. Cathode power-feeding shaft 426 is connected to power source 7 by wiring. power source 7 can apply driving voltage between anode 42c and cathode 42d using anode power-feeding shaft 425 and cathode power-feeding shaft 426.

When power source 7 applies driving voltage between anode 42c and cathode 42d in a state where tap water W1 flows from upstream connector 423 into recess 422, a potential difference is generated between anode 42c and cathode 42d via conductive film 42e provided therebetween. When a potential difference is generated between anode 42c and cathode 42d, anode 42c, conductive film 42e, and cathode 42d are energized, and a driving current flows from anode 42c toward cathode 42d. When the driving current flows, tap water W1 is electrolyzed to generate ozone near an interface between conductive film 42e and anode 42c. The ozone is dissolved in tap water W1 while being carried toward downstream connector 424 along a flow of tap water W1 in recess 422, thereby generating ozone water W2. The driving voltage applied between anode 42c and cathode 42d by power source 7 is several volts to several tens of volts, and the amount of generation of ozone increases as the driving current increases (as the driving voltage increases). The driving current is fed from power source 7 to electrolytic part 42 for electrolysis.

As described above, electrolytic part 42 generates ozone water W2 (electrolytic liquid) by dissolving ozone (electrolytic product) in tap water W1. Electrolytic part 42 decreases bacteria contained in tap water W1 by oxidation action of ozone to generate ozone water W2 having less bacteria.

Electrolytic part 42 can adjust the amount of ozone generated by electrolytic part 42 (the amount of ozone contained in ozone water W2) by adjusting the driving current. Electrolytic part 42 is notified of an instruction value (current instruction value) of the driving current by current instruction signal S2 from controller 5 to adjust the driving current so that the driving current has a value equal to the current instruction value. That is, functional unit 4 can increase the amount of ozone contained in ozone water W2 by increasing the driving current to increase the amount of ozone generated by electrolytic part 42, and as a result, the disinfection function can be improved. Alternatively, functional unit 4 can decrease the amount of ozone contained in ozone water W2 by decreasing the driving current to decrease the amount of ozone generated by electrolytic part 42, and as a result, the disinfection function can be deteriorated.

### (1.1.4) Liquid outflow path

Liquid outflow path 32 is a water pipe such as a hose, a resin pipe, or a metal pipe. Liquid outflow path 32 has an inflow port connected to downstream connector 424 (see Fig. 2) of electrolytic part 42, and ozone water W2 flows from electrolytic part 42 into liquid outflow path 32. Liquid outflow path 32 is connected to water feed port 6, and ozone water W2 flowing through liquid outflow path 32 is fed from water feed port 6 to the outside of electrolytic liquid generation system 1.

### (1.1.5) Water feed port

Water feed port 6 allows ozone water W2 flowing through liquid outflow path 32 to be fed to the outside of electrolytic liquid generation system 1.

For example, when electrolytic liquid generation system 1 is incorporated in a water purifier, water feed port 6 constitutes a water discharge port of the water purifier, and ozone water W2 is discharged from water feed port 6. When electrolytic liquid generation system 1 is incorporated in a mist generator, the mist generator generates a mist using ozone water W2 fed from water feed port 6, and sprays the generated mist.

### (1.1.6) Power source

Power source 7 applies driving voltage to electrolytic part 42 to generate a potential difference between anode 42c and cathode 42d, thereby generating a driving current from anode 42c to cathode 42d. That is, power source 7 applies driving voltage to electrolytic part 42, and supplies a driving current to electrolytic part 42.

### (1.1.7) Detector

Detector 8 detects a degree of contamination of at least one of tap water W1 or ozone water W2. Water having a lower degree of contamination is considered to be clean and harmless.

Detector 8 in the present exemplary embodiment is upstream detector 81 that detects a degree of contamination of tap water W1 flowing through liquid inflow path 31. Upstream detector 81 defines the number of bacteria contained in tap water W1, turbidity, color, organic substance concentration, or residence time in liquid inflow path 31, of tap water W1, for example, as a first concentration detection value representing the degree of contamination of tap water W1. Upstream detector 81 may define a reciprocal of residual chlorine concentration of tap water W1 as the first contamination detection value. The first contamination detection value decreases with a decrease in the number of bacteria in tap water W1, a decrease in the turbidity, an increase in transparency in the color, a decrease in the organic substance concentration, a decrease in the residence time in liquid inflow path 31, of tap water W1, or a decrease in the reciprocal of the residual chlorine concentration of tap water W1. As the first concentration detection value described above decreases, the degree of contamination of tap water W1 decreases.

Upstream detector 81 outputs detection signal S3 notifying controller 5 of a first contamination detection value. Controller 5 determines the degree of contamination of tap water W1 based on the first contamination detection value included in detection signal S3, and controls functional unit 4 based on the determination result.

Detector 8 includes: a sensor that detects a degree of contamination of a liquid, such as a bacteria count sensor, a turbidity sensor, a chromaticity sensor, or an organic substance concentration sensor; a sensor that detects concentration of a substance having a disinfection effect, such as chlorine or ozone, remaining in the liquid, such as a residual chlorine concentration sensor or an ozone concentration sensor; or a flow rate sensor, for example.

### (1.1.8) Controller

Controller 5 includes a computer system. That is, controller 5 implements some or all of its functions by causing a processor such as a central processing unit (CPU) or a micro processing unit (MPU) to read out and execute a program stored in a memory. Controller 5 includes a processor that operates according to a program as a main hardware configuration. The processor is regardless of type as long as the processor can implement the functions by executing the program. The processor includes one or more electronic circuits including a semiconductor integrated circuit (IC) or a large scale integration (LSI). Although the processor is referred to as the IC or the LSI herein, the terms vary depending on a degree of integration, and may be referred to as a system LSI, a very large scale integration (VLSI), or an ultra large scale integration (ULSI). A field-programmable gate array (FPGA) that is programmed after fabrication of the LSI, or a reconfigurable logic device capable of reconfiguring a joint relationship inside the LSI or setting up a circuit section inside the LSI can also be used for the same purpose. The electronic circuits may be integrated on one chip or may be provided on respective chips. The chips may be collectively disposed or may be disposed in a distributed manner.

Specifically, controller 5 includes flow rate controller 51 and current controller 52.

When controller 5 receives detection signal S3 from upstream detector 81, flow rate controller 51 determines a degree of contamination of tap water W1 based on detection signal S3, and outputs flow rate instruction signal S1 based on the determination result to flow rate adjuster 41 of functional unit 4 to control flow rate adjuster 41. That is, flow rate controller 51 controls a liquid feed rate (a flow rate of tap water W1 fed to electrolytic part 42) by controlling flow rate adjuster 41. For example, flow rate controller 51 can increase the amount of generation of ozone water W2 by increasing the liquid feed rate using flow rate adjuster 41, and as a result, the electrolytic liquid generation function can be improved. Alternatively, flow rate controller 51 can decrease the amount of generation of ozone water W2 by decreasing the liquid feed rate using flow rate adjuster 41, and as a result, the electrolytic liquid generation function can be deteriorated.

When controller 5 receives detection signal S3 from upstream detector 81, current controller 52 determines a degree of contamination of tap water W1 based on detection signal S3, and outputs current instruction signal S2 based on the determination result to electrolytic part 42 of functional unit 4 to control electrolytic part 42. That is, current controller 52 controls electrolytic part 42 to control the amount of ozone generated by electrolytic part 42. For example, current controller 52 can increase the amount of ozone contained in ozone water W2 by increasing the driving current of electrolytic part 42 to increase the amount of ozone generated by electrolytic part 42, and as a result, the disinfection function can be improved. Alternatively, current controller 52 can decrease the amount of ozone contained in ozone water W2 by decreasing the driving current of electrolytic part 42 to decrease the amount of ozone generated by electrolytic part 42, and as a result, the disinfection function can be deteriorated. Hereinafter, the total amount of ozone generated by electrolytic part 42 may be referred to as a total amount of generation of ozone.

### (1.2) Operation

Controller 5 operates in one of multiple control modes. The multiple control modes include a first control mode and a second control mode. Controller 5 improves the electrolytic liquid generation function in the first control mode more than in the second control mode. Controller 5 improves the disinfection function in the second control mode more than in the first control mode. That is, controller 5 gives priority to the electrolytic liquid generation function in the first control mode, and gives priority to the disinfection function in the second control mode. As a result, electrolytic liquid generation system 1 can adjust balance between disinfection of tap water W1 and generation efficiency of ozone water W2.

Controller 5 selects any one of the multiple control modes according to a degree of contamination of tap water W1, and operates in the selected control mode. Controller 5 operates in the first control mode when the degree of contamination of tap water W1 is less than a mode threshold, and operates in the second control mode when the degree of contamination of tap water W1 is equal to or greater than the mode threshold.

Controller 5 in the present exemplary embodiment receives detection signal S3 from upstream detector 81 as a detection result of the degree of contamination of tap water W1, detection signal S3 including the first contamination detection value such as the number of bacteria contained in tap water W1, turbidity, color, organic substance concentration, or residence time in liquid inflow path 31, of tap water W1, or the reciprocal of residual chlorine concentration of tap water W1. Then, controller 5 compares the first contamination detection value with the mode threshold, and operates in the first control mode when the first contamination detection value is less than the mode threshold, and operates in the second control mode when the first contamination detection value is equal to or greater than the mode threshold. That is, electrolytic liquid generation system 1 can automatically switch the control mode based on the degree of contamination of tap water W1.

In particular, after electrolytic part 42 generates ozone water W2, the ozone contained in ozone water W2 decreases as time elapses, and thus the disinfection effect in ozone water W2 does not continue. Thus, when electrolytic liquid generation system 1 is stopped, bacteria contained in ozone water W2 remaining in liquid outflow path 32 may increase. For this reason, upstream detector 81 preferably detects the residence time of tap water W1 in liquid inflow path 31 as the first contamination detection value. The residence time of tap water W1 is equal to stop time of electrolytic liquid generation system 1. When electrolytic liquid generation system 1 that has not been used for a long period of time starts operating, the residence time of tap water W1 becomes equal to or longer than a predetermined time. Then, controller 5 automatically operates in the second control mode to give priority to the disinfection function, so that disinfection efficiency can be improved.

Hereinafter, an operation example of electrolytic liquid generation system 1 of the present exemplary embodiment will be described.

### (1.2.1) First operation example

Electrolytic liquid generation system 1 operates as follows in a first operation example.

Controller 5 operates in the first control mode when the first contamination detection value is less than the mode threshold.

When controller 5 operates in the first control mode, flow rate controller 51 controls flow rate adjuster 41 to set a liquid feed rate (a flow rate of tap water W1 fed to electrolytic part 42) to be F1.

When controller 5 operates in the first control mode, current controller 52 controls electrolytic part 42 to set the driving current of electrolytic part 42 to be I1.

Controller 5 operates in the second control mode when the first contamination detection value is equal to or larger than the mode threshold.

When controller 5 operates in the second control mode, flow rate controller 51 controls flow rate adjuster 41 to set the liquid feed rate to be F2 (< F1). That is, liquid feed rate F2 in the second control mode is smaller than liquid feed rate F1 in the first control mode.

When controller 5 operates in the second control mode, current controller 52 controls electrolytic part 42 to set the driving current of electrolytic part 42 to be I2 (> I1). That is, driving current I2 in the second control mode is larger than driving current I1 in the first control mode. As a result, the total amount of generation of ozone in the second control mode is larger than the total amount of generation of ozone in the first control mode.

Thus, the amount of ozone water W2 generated in the second control mode is smaller than the amount of ozone water W2 generated in the first control mode. Then, ozone concentration of ozone water W2 generated in the second control mode is larger than ozone concentration of ozone water W2 generated in the first control mode. That is, electrolytic liquid generation system 1 can increase the disinfection efficiency and save tap water W1 in the second control mode.

In other words, controller 5 improves the electrolytic liquid generation function in the first control mode more than in the second control mode, and deteriorates the disinfection function in the first control mode more than in the second control mode. Controller 5 also improves the disinfection function in the second control mode more than in the first control mode, and deteriorates the electrolytic liquid generation function in the second control mode more than in the first control mode.

Flow rate controller 51 preferably sets liquid feed rate F2 in the second control mode to a liquid feed rate equal to or less than 1/2 of liquid feed rate F1 in the first control mode and larger than 0 (zero). Specifically, when liquid feed rate F1 is 1.0 L (liter)/min (minute), for example, liquid feed rate F2 may be set to 0.5 L/min or less. In this case, electrolytic liquid generation system 1 can increase the disinfection efficiency and save tap water W1.

More preferably, flow rate controller 51 may set liquid feed rate F2 in the second control mode to 1/5 or less of liquid feed rate F1 in the first control mode. Specifically, when liquid feed rate F1 is 1.0 L/min, for example, liquid feed rate F2 may be set to 0.2 L/min or less. In this case, electrolytic liquid generation system 1 can further increase the disinfection efficiency and further save tap water W1.

More preferably, flow rate controller 51 may set liquid feed rate F2 in the second control mode to 1/8 or less of liquid feed rate F1 in the first control mode. Specifically, when liquid feed rate F1 is 1.0 L/min, for example, liquid feed rate F2 may be set to 0.125 L/min or less. In this case, electrolytic liquid generation system 1 can further increase the disinfection efficiency and further save tap water W1.

Liquid feed rate F2 in the second control mode may be less than liquid feed rate F1 in the first control mode and larger than 1/2 of liquid feed rate F1.

### (1.2.2) Second operation example

Electrolytic liquid generation system 1 operates as follows in a second operation example.

Controller 5 operates in the first control mode when the first contamination detection value is less than the mode threshold.

When controller 5 operates in the first control mode, flow rate controller 51 controls flow rate adjuster 41 to set a liquid feed rate (a flow rate of tap water W1 fed to electrolytic part 42) to be F1.

When controller 5 operates in the first control mode, current controller 52 controls electrolytic part 42 to set the driving current of electrolytic part 42 to be I1.

Controller 5 operates in the second control mode when the first contamination detection value is equal to or larger than the mode threshold.

When controller 5 operates in the second control mode, flow rate controller 51 controls flow rate adjuster 41 to set the liquid feed rate to be F3 (< F1). That is, liquid feed rate F3 in the second control mode is smaller than liquid feed rate F1 in the first control mode.

When controller 5 operates in the second control mode, current controller 52 controls electrolytic part 42 to set the driving current of electrolytic part 42 to be I3 (< I1). That is, driving current I3 in the second control mode is smaller than driving current I1 in the first control mode. As a result, the total amount of generation of ozone in the second control mode is smaller than the total amount of generation of ozone in the first control mode. However, liquid feed rate F3 in the second control mode is smaller than liquid feed rate F1 in the first control mode. For this reason, a value of driving current I3 is preliminarily set such that ozone concentration of ozone water W2 generated in the second control mode is larger than ozone concentration of ozone water W2 generated in the first control mode.

Thus, the amount of ozone water W2 generated in the second control mode is smaller than the amount of ozone water W2 generated in the first control mode. Then, ozone concentration of ozone water W2 generated in the second control mode is larger than ozone concentration of ozone water W2 generated in the first control mode. That is, electrolytic liquid generation system 1 can increase the disinfection efficiency and save tap water W1 in the second control mode.

In other words, controller 5 improves the electrolytic liquid generation function in the first control mode more than in the second control mode, and deteriorates the disinfection function in the first control mode more than in the second control mode. Controller 5 also improves the disinfection function in the second control mode more than in the first control mode, and deteriorates the electrolytic liquid generation function in the second control mode more than in the first control mode.

Electrolytic liquid generation system 1 can achieve a long life of electrolytic part 42 by decreasing driving current I3 in the second control mode to smaller than driving current I1 in the first control mode to suppress deterioration of electrolytic part 42 in the second control mode.

Current controller 52 preferably sets driving current I3 in the second control mode to a current value that is 1/2 or less of driving current I1 in the first control mode and is larger than 0 (zero). In this case, suppression of deterioration of electrolytic part 42 and a long life of electrolytic part 42 in the second control mode can be reliably achieved.

Driving current I3 in the second control mode may be 1/5 or less, or 1/8 or less of driving current I1 in the first control mode.

Driving current I3 in the second control mode may be less than driving current I1 in the first control mode and greater than 1/2 of driving current I1.

### (1.2.3) Third operation example

Electrolytic liquid generation system 1 operates as follows in a third operation example.

Controller 5 operates in the first control mode when the first contamination detection value is less than the mode threshold.

When controller 5 operates in the first control mode, flow rate controller 51 controls flow rate adjuster 41 to set a liquid feed rate (a flow rate of tap water W1 fed to electrolytic part 42) to be F1.

When controller 5 operates in the first control mode, current controller 52 controls electrolytic part 42 to set the driving current of electrolytic part 42 to be I1.

Controller 5 operates in the second control mode when the first contamination detection value is equal to or larger than the mode threshold.

When controller 5 operates in the second control mode, flow rate controller 51 controls flow rate adjuster 41 to set a liquid feed rate equal to F1 in the first control mode. That is, the liquid feed rate does not change in the first control mode and the second control mode.

When controller 5 operates in the second control mode, current controller 52 controls electrolytic part 42 to set the driving current of electrolytic part 42 to be I4 (> I1). Driving current I4 in the second control mode is larger than driving current I1 in the first control mode, so that the total amount of generation of ozone in the second control mode is larger than the total amount of generation of ozone in the first control mode.

Thus, the amount of ozone water W2 generated in the second control mode is equal to the amount of ozone water W2 generated in the first control mode. However, the total amount of generation of ozone in the second control mode is larger than the total amount of generation of ozone in the first control mode, so that the ozone concentration of ozone water W2 generated in the second control mode is larger than the ozone concentration of ozone water W2 generated in the first control mode. That is, electrolytic liquid generation system 1 can increase the disinfection efficiency in the second control mode.

In other words, controller 5 deteriorates the disinfection function in the first control mode more than in the second control mode. Controller 5 improves the disinfection function in the second control mode more than in the first control mode.

Current controller 52 preferably sets driving current I4 in the second control mode to 4/3 or more of driving current I1 in the first control mode. In this case, the disinfection efficiency in the second control mode can be sufficiently increased.

### (1.2.4) Fourth operation example

Electrolytic liquid generation system 1 operates as follows in a fourth operation example.

Controller 5 operates in the first control mode when the first contamination detection value is less than the mode threshold.

When controller 5 operates in the first control mode, flow rate controller 51 controls flow rate adjuster 41 to set a liquid feed rate (a flow rate of tap water W1 fed to electrolytic part 42) to be F1.

When controller 5 operates in the first control mode, current controller 52 controls electrolytic part 42 to set the driving current of electrolytic part 42 to be I1.

Controller 5 operates in the second control mode when the first contamination detection value is equal to or larger than the mode threshold.

When controller 5 operates in the second control mode, flow rate controller 51 controls flow rate adjuster 41 to set the liquid feed rate to be F4 (< F1). That is, liquid feed rate F4 in the second control mode is smaller than liquid feed rate F1 in the first control mode.

When controller 5 operates in the second control mode, current controller 52 controls electrolytic part 42 to set the driving current of electrolytic part 42 to be I1 equal in the first control mode. That is, the driving current does not change in the first control mode and the second control mode. However, liquid feed rate F4 in the second mode is smaller than liquid feed rate F1 in the first mode, so that the ozone concentration of ozone water W2 generated in the second control mode is larger than the ozone concentration of ozone water W2 generated in the first control mode. That is, electrolytic liquid generation system 1 can increase the disinfection efficiency and save tap water W1 in the second control mode.

In other words, controller 5 improves the electrolytic liquid generation function in the first control mode more than in the second control mode, and deteriorates the disinfection function in the first control mode more than in the second control mode. Controller 5 also improves the disinfection function in the second control mode more than in the first control mode, and deteriorates the electrolytic liquid generation function in the second control mode more than in the first control mode.

### (1.3) First modification

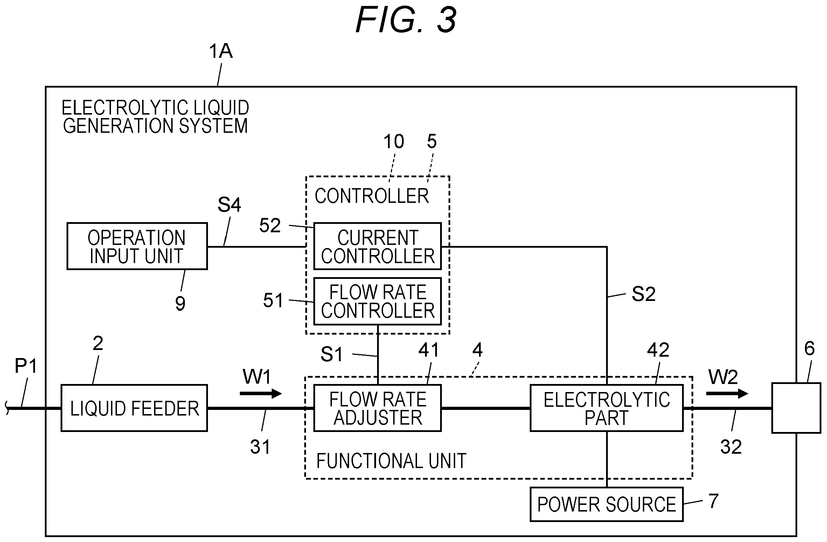

Fig. 3 shows a block diagram of electrolytic liquid generation system 1A as a first modification of electrolytic liquid generation system 1.

Electrolytic liquid generation system 1A includes operation input unit 9 instead of detector 8 (upstream detector 81) of electrolytic liquid generation system 1.

Operation input unit 9 includes at least one of a switch, a button, a touch panel, or a receiver. The switch, the button, and the touch panel directly receive a user's operation. The receiver indirectly receives a user's operation by receiving a wireless signal or a wired signal from a remote controller operated by the user.

The user's operation received by operation input unit 9 includes a mode selection operation for selecting a control mode. That is, the user can instruct controller 5 whether to operate controller 5 in the first control mode or the second control mode by operating operation input unit 9.

In Fig. 3, operation input unit 9 outputs operation signal S4 corresponding to a user's operation to controller 5. Operation signal S4 instructs controller 5 on a control mode. When the control mode instructed by operation signal S4 is the first control mode, controller 5 operates in the first control mode. When the control mode instructed by operation signal S4 is the second control mode, controller 5 operates in the second control mode. That is, electrolytic liquid generation system 1A can switch the control mode by a user's operation.

Each operation of flow rate controller 51 and current controller 52 of controller 5 in each of the first control mode and the second control mode is the same as any of the first operation example to the fourth operation example described above, and thus the description thereof will be omitted.

For example, when electrolytic liquid generation system 1A that has not been used for a long period of time is used, the user operates operation input unit 9 at the start of use to select the second control mode. Thus, when electrolytic liquid generation system 1A that has not been used for a long period of time starts operating, controller 5 operates in the second control mode by a user's operation to give priority to the disinfection function and thus enables improving the disinfection efficiency.

As illustrated in Fig. 1, electrolytic liquid generation system 1 may further include operation input unit 9 of the first modification. In this case, electrolytic liquid generation system 1 also allows controller 5 to operate in the second control mode by a user's operation to give priority to the disinfection function, so that the disinfection efficiency can be improved.

### (1.4) Second modification

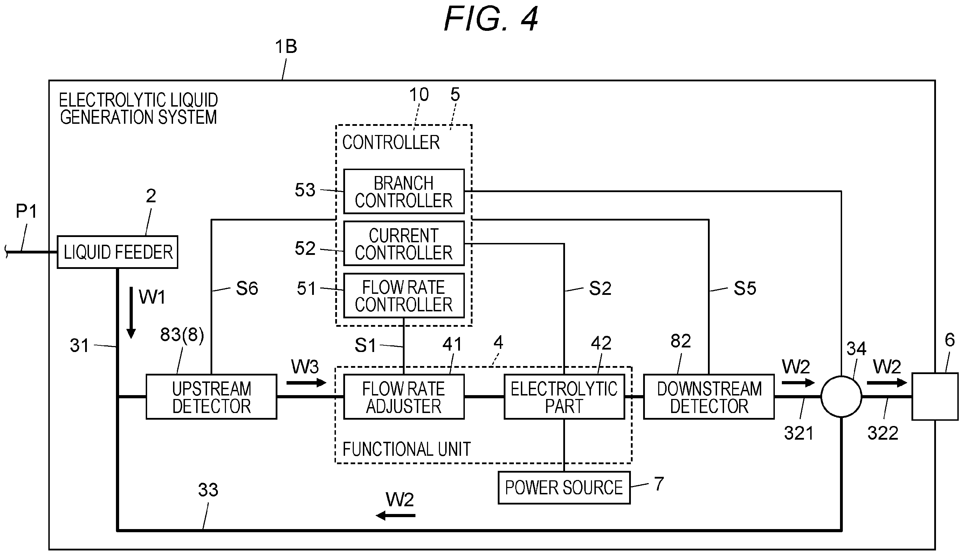

Fig. 4 shows a block diagram of electrolytic liquid generation system 1B as a second modification of electrolytic liquid generation system 1. Electrolytic liquid generation system 1B includes liquid outflow paths 321, 322 into which liquid outflow path 32 of electrolytic liquid generation system 1 in Fig. 1 is divided, and further includes circulation flow path 33 and branch part 34. Controller 5 of electrolytic liquid generation system 1B further includes branch controller 53. Electrolytic liquid generation system 1B further includes downstream detector 82 and upstream detector 83.

Electrolytic liquid generation system 1B allows electrolytic part 42 to feed generated ozone water W2 to liquid outflow path 321. Liquid outflow path 321 includes branch part 34. Branch part 34 includes an electromagnetic valve, and has a function of selectively branching ozone water W2 from liquid outflow path 321 to any one of liquid outflow path 322 and circulation flow path 33. That is, branch part 34 connects liquid outflow path passage 321 to any one of liquid outflow path 322 and circulation flow path 33 in a switchable manner. Liquid outflow path 322 is connected to water feed port 6, and allows ozone water W2 fed through branch part 34 to be fed to water feed port 6. Circulation flow path 33 is connected to liquid inflow path 31, and allows ozone water W2 fed through branch part 34 to be returned to liquid inflow path 31. Branch controller 53 controls branch part 34 to switch liquid outflow path 321 to any one of liquid outflow path 322 and circulation flow path 33.

Downstream detector 82 detects the degree of contamination of ozone water W2 flowing through liquid outflow path 321. Downstream detector 82 defines the number of bacteria contained in ozone water W2, turbidity, color, organic substance concentration, or residence time in liquid outflow path 321, of ozone water W2, for example, as a second concentration detection value representing the degree of contamination of ozone water W2. Downstream detector 82 may define a reciprocal of residual chlorine concentration of ozone water W2 as the second contamination detection value. The second contamination detection value decreases with a decrease in the number of bacteria in ozone water W2, a decrease in the turbidity, an increase in transparency in the color, a decrease in the organic substance concentration, a decrease in the residence time in liquid outflow path 321, of ozone water W2, or a decrease in the reciprocal of the residual chlorine concentration of ozone water W2. As the second concentration detection value decreases, the degree of contamination of ozone water W2 decreases.

Downstream detector 82 outputs detection signal S5 notifying controller 5 of a second contamination detection value. Controller 5 determines the degree of contamination of ozone water W2 based on the second contamination detection value included in detection signal S5, and branch controller 53 controls branch part 34 based on the determination result.

Specifically, branch controller 53 compares the second contamination detection value with a branch threshold. When the second contamination detection value is less than the branch threshold, branch controller 53 controls branch part 34 to connect liquid outflow path 321 to liquid outflow path 322. When liquid outflow path 321 is connected to liquid outflow path 322, ozone water W2 generated by electrolytic part 42 is fed to water feed port 6 through liquid outflow path 322.

When the second contamination detection value is equal to or larger than the branch threshold, branch controller 53 controls branch part 34 to connect liquid outflow path 321 to circulation flow path 33. When liquid outflow path 321 is connected to circulation flow path 33, ozone water W2 generated by electrolytic part 42 flows through circulation flow path 33 and is fed to liquid inflow path 31. Then, tap water W1 and ozone water W2 are mixed in liquid inflow path 31. Mixed liquid W3 in which tap water W1 and ozone water W2 are mixed is fed to functional unit 4 through upstream detector 81. Functional unit 4 electrolyzes mixed liquid W3 to perform the electrolytic liquid generation function of generating ozone water W2 and the disinfection function of disinfecting mixed liquid W3. In this case, mixed liquid W3 corresponds to a liquid.

As described above, ozone water W2 having an insufficiently low degree of contamination is disinfected again by electrolytic part 42 to prevent ozone water W2 having the insufficiently low degree of contamination from flowing out from water feed port 6. Thus, ozone water W2 having a sufficiently low degree of contamination is fed from water feed port 6, so that ozone water W2 can be improved in quality.

Upstream detector 83 serves as detector 8 of electrolytic liquid generation system 1B. Upstream detector 83 detects a degree of contamination of mixed liquid W3. Upstream detector 83 defines the number of bacteria contained in mixed liquid W3, turbidity, color, organic substance concentration, or residence time of mixed liquid W3, for example, as a third concentration detection value representing the degree of contamination of mixed liquid W3. Upstream detector 83 may define a reciprocal of residual chlorine concentration of mixed liquid W3 as the third contamination detection value. The third contamination detection value decreases with a decrease in the number of bacteria in mixed liquid W3, a decrease in the turbidity, an increase in transparency in the color, a decrease in the organic substance concentration, a decrease in the residence time, of mixed liquid W3, or a decrease in the reciprocal of the residual chlorine concentration of mixed liquid W3. As the third concentration detection value decreases, the degree of contamination of mixed liquid W3 decreases.

Upstream detector 83 outputs detection signal S6 notifying controller 5 of a third contamination detection value. Controller 5 determines the degree of contamination of mixed liquid W3 based on the third contamination detection value included in detection signal S6, and controls functional unit 4 based on the determination result.

Controller 5 then compares the third contamination detection value with the mode threshold, and operates in the first control mode when the third contamination detection value is less than the mode threshold, and operates in the second control mode when the third contamination detection value is equal to or greater than the mode threshold. That is, electrolytic liquid generation system 1B can automatically switch the control mode based on the degree of contamination of mixed liquid W3.

Each operation of flow rate controller 51 and current controller 52 of controller 5 in each of the first control mode and the second control mode is the same as any of the first operation example to the fourth operation example described above, and thus the description thereof will be omitted.

Electrolytic liquid generation system 1B of the second modification is preferably used particularly in a mist cooler (mist generator). The mist cooler ejects a mist in which ozone water W2 is formed into fine particles, and takes away ambient heat of vaporization to lower ambient temperature. In this case, the mist is sufficiently disinfected, and improved in safety.

### (1.5) Third modification

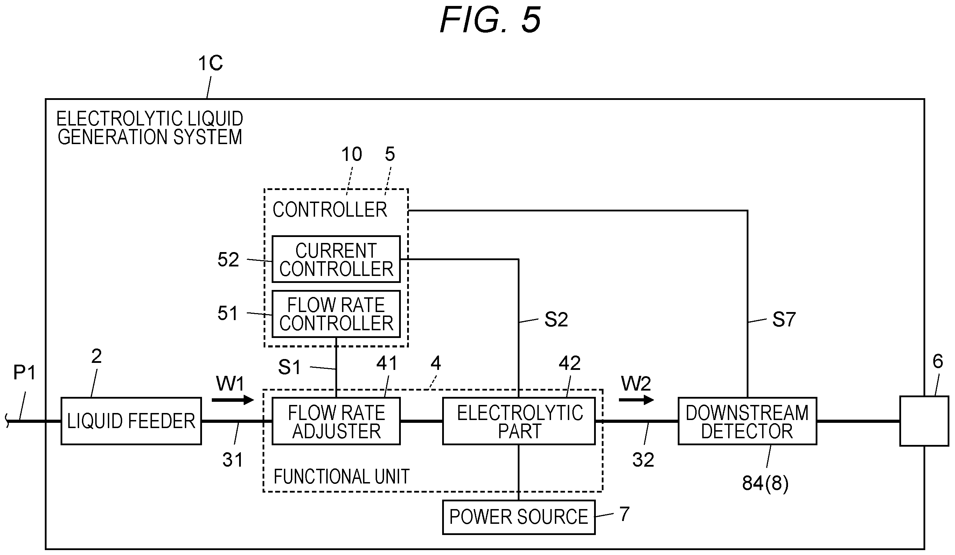

Fig. 5 shows a block diagram of electrolytic liquid generation system 1C as a third modification of electrolytic liquid generation system 1.

Electrolytic liquid generation system 1C includes downstream detector 84 as detector 8, instead of upstream detector 81 of electrolytic liquid generation system 1.

Downstream detector 84 detects the degree of contamination of ozone water W2 flowing through liquid outflow path 32. Downstream detector 84 defines the number of bacteria contained in ozone water W2, turbidity, color, organic substance concentration, or residence time in liquid outflow path 32, of ozone water W2, for example, as a fourth concentration detection value representing the degree of contamination of ozone water W2. Downstream detector 84 may define a reciprocal of residual chlorine concentration of ozone water W2 or a reciprocal of ion concentration of ozone water W2 as the fourth contamination detection value. The fourth contamination detection value decreases with a decrease in the number of bacteria in ozone water W2, a decrease in the turbidity, an increase in transparency in the color, a decrease in the organic substance concentration, a decrease in the residence time in liquid outflow path 32, of ozone water W2, a decrease in the reciprocal of the residual chlorine concentration of ozone water W2, or a decrease in the reciprocal of ion concentration of ozone water W2. As the fourth concentration detection value decreases, the degree of contamination of ozone water W2 decreases.

Downstream detector 84 outputs detection signal S7 notifying controller 5 of a fourth contamination detection value. Controller 5 then compares the fourth contamination detection value with the mode threshold, and operates in the first control mode when the fourth contamination detection value is less than the mode threshold, and operates in the second control mode when the fourth contamination detection value is equal to or greater than the mode threshold. That is, electrolytic liquid generation system 1C can automatically switch the control mode based on the degree of contamination of ozone water W2.

Each operation of flow rate controller 51 and current controller 52 of controller 5 in each of the first control mode and the second control mode is the same as any of the first operation example to the fourth operation example described above, and thus the description thereof will be omitted.

### (2) Second exemplary embodiment

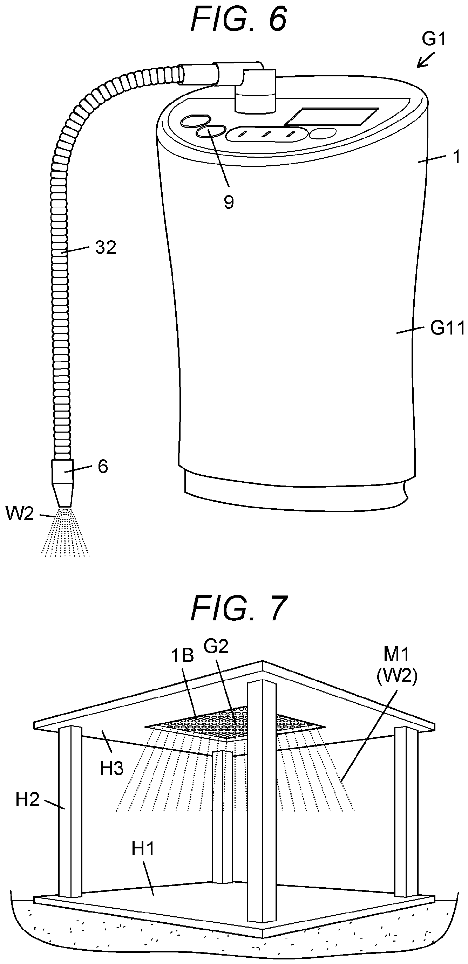

Fig. 6 illustrates an appearance of water purifier G1 as an example of a device using electrolytic liquid generation system 1 described above.

Water purifier G1 includes body G11 in a hollow cylindrical shape, and a part of electrolytic liquid generation system 1 is housed in body G11. Liquid outflow path 32 formed of a flexible hose extends from body G11, and water feed port 6 serving as a water discharge port is attached to a leading end of liquid outflow path 32.

Water purifier G1 may include electrolytic liquid generation system 1A, 1B, or 1C instead of electrolytic liquid generation system 1.

### (3) Third exemplary embodiment

Fig. 7 illustrates an appearance of mist cooler G2 as another example of a device using electrolytic liquid generation system 1B described above.

Mist cooler G2 is attached to an outdoor structure. Fig. 7 illustrates a structure including base H1 formed on the ground, four columns H2 installed on base H1, and ceiling material H3 supported above base H1 by four columns H2. Mist cooler G2 is attached to a lower surface of ceiling material H3, and ejects mist M1 in which ozone water W2 is formed into fine particles downward to take away ambient heat of vaporization, thereby lowering ambient temperature.

Mist cooler G2 may include electrolytic liquid generation system 1, 1A, or 1C instead of electrolytic liquid generation system 1B.

### (4) Other modifications

Each of electrolytic liquid generation systems 1, 1A, 1B, and 1C of the present disclosure adjusts a balance between disinfection of tap water W1 and generation efficiency of ozone water W2, and an operation for adjusting the balance is not limited to each operation described above. That is, as long as each of electrolytic liquid generation systems 1, 1A, 1B, and 1C can satisfactorily utilize the disinfection effect of tap water W1, the operation for adjusting the balance between the disinfection of tap water W1 and the generation efficiency of ozone water W2 is not limited to a specific operation.

In addition to the first control mode and the second control mode, the control mode may further include a third mode in which tap water W1 is fed from water feed port 6 without performing the electrolytic treatment in electrolytic part 42.

In addition to the first control mode and the second control mode, the control mode may further include a strong mode for enhancing the disinfection function and a weak mode for lowering the disinfection function.

Controller 5 may be configured to control at least one of the electrolytic liquid generation function or the disinfection function of functional unit 4. Controller 5 may be configured to control only one of flow rate adjuster 41 and electrolytic part 42.

The liquid is not limited to tap water W1, and may be another liquid. Electrolytic liquid is not limited to ozone water W2, and may be another liquid having a disinfection effect. For example, the liquid may be salt water, and the electrolytic liquid may be electrolytic hypo-water.

The liquid is not limited to a liquid fed from waterworks P1, and may be a liquid flowing inside a device, an appliance, or the like.

The electrolytic liquid generation system 1 may have a system configuration in which liquid feeder 2, liquid inflow path 31, liquid outflow path 32, functional unit 4, controller 5, water feed port 6, power source 7, and detector 8 are dispersed in two or more devices.

A function similar to that of control system 10 composed of controller 5 described above may be embodied by a control method of each of electrolytic liquid generation systems 1, 1A, 1B, and 1C, a computer program, or a recording medium in which the computer program is recorded. That is, the function of control system 10 may be embodied by the control method of each of electrolytic liquid generation systems 1, 1A, 1B, and 1C, the computer program, the recording medium recording the computer program, or the like.

The term "disinfection" described above may be replaced with "sterilization" or "sterilizing". The "disinfection" means that bacteria are decreased by eliminating them. The "sterilization" can be used for a "pharmaceutical product" or a "quasi-pharmaceutical product". The "sterilizing" is defined by the Japanese Pharmacopoeia as "probability of surviving of a microorganism being 1/1 million or less".

### (5) Summary

Electrolytic liquid generation system (1, 1A, 1B, 1C) of a first aspect according to the corresponding one of the exemplary embodiments described above includes liquid inflow path (31), functional unit (4), and controller (5). Liquid inflow path (31) allows liquid (W1, W3) to flows therethrough. Functional unit (4) electrolyzes liquid (W1, W3) fed from liquid inflow path (31) to perform the disinfection function of disinfecting liquid (W1, W3) while performing the electrolytic liquid generation function of generating electrolytic liquid (W2) from liquid (W1, W3). Controller (5) controls at least one of the electrolytic liquid generation function or the disinfection function of functional unit (4). Controller (5) operates in any of the multiple control modes including at least the first control mode and the second control mode. Controller (5) improves the electrolytic liquid generation function in the first control mode more than in the second control mode. Controller (5) improves the disinfection function in the second control mode more than in the first control mode.

Electrolytic liquid generation system (1, 1A, 1B, 1C) described above can adjust a balance between disinfection of liquid (W1, W3) and generation efficiency of electrolytic liquid (W2).

In electrolytic liquid generation system (1, 1A, 1B, 1C) of a second aspect according to the corresponding one of the exemplary embodiments described above, in the first aspect, it is preferable that functional unit (4) includes flow rate adjuster (41) configured to adjust a liquid feed rate that is the amount of liquid (W1, W3) fed from liquid inflow path (31). Controller (5) controls the electrolytic liquid generation function by controlling flow rate adjuster (41) to decrease the liquid feed rate more in the second control mode than in the first control mode.

Electrolytic liquid generation system (1, 1A, 1B, 1C) can increase the disinfection efficiency and save liquid (W1, W3) in the second control mode.

In electrolytic liquid generation system (1, 1A, 1B, 1C) of a third aspect according to the corresponding one of the exemplary embodiments described above, in the first or second aspect, it is preferable that functional unit (4) includes electrolytic part (42) configured to electrolyze liquid (W1, W3). Controller (5) controls a current (driving current) to be fed to electrolytic part (42) to control the disinfection function by increase the current in the second control mode to more than the current in the first control mode.

Electrolytic liquid generation system (1, 1A, 1B, 1C) described above can increase the disinfection efficiency in the second control mode.

In electrolytic liquid generation system (1, 1A, 1B, 1C) of a fourth aspect according to the corresponding one of the exemplary embodiments described above, in the second aspect, it is preferable that functional unit (4) further includes electrolytic part (42) configured to electrolyze liquid (W1, W3). Controller (5) controls a current (driving current) to be fed to electrolytic part (42) to control the disinfection function by decreasing the current in the second control mode to less than the current in the first control mode.

Electrolytic liquid generation system (1, 1A, 1B, 1C) described above can suppress deterioration of electrolytic part (42) in the second control mode to achieve a long life of electrolytic part (42).

Electrolytic liquid generation system (1A) of a fifth aspect according to the corresponding one of the exemplary embodiments described above, in any one of the first to fourth aspects, preferably further includes operation input unit (9) configured to receive a human operation. Controller (5) selects any one of the multiple control modes in response to the operation received by operation input unit (9), and operates in the selected control mode.

Electrolytic liquid generation system (1A) described above enables controller (5) to switch a control mode by a user's operation.

Electrolytic liquid generation system (1, 1B, 1C) of a sixth aspect according to the corresponding one of the exemplary embodiments described above, in any one of the first to fourth aspects, preferably further includes detector (8) configured to detect a degree of contamination of at least one of liquid (W1, W3) or electrolytic liquid (W2). Controller (5) selects any one of the multiple control modes in accordance with the degree of contamination, and operates in the selected control mode.

Electrolytic liquid generation system (1, 1B, 1C) described above can automatically switch the control mode based on the degree of contamination of at least one of liquid (W1, W3) or electrolytic liquid (W2).

In electrolytic liquid generation system (1, 1B, 1C) of a seventh aspect according to the corresponding one of the exemplary embodiments described above, in the sixth aspect, it is preferable that controller (5) is configured to operate in the first control mode when the degree of contamination is less than the mode threshold, and to operate in the second control mode when the degree of contamination is equal to or more than the mode threshold.

The electrolytic liquid generation system (1, 1B, 1C) described above can automatically improve the disinfection function when the degree of contamination of at least one of liquid (W1, W3) or electrolytic liquid (W2) is equal to or more than the mode threshold value.

In electrolytic liquid generation system (1, 1A, 1B, 1C) of an eighth aspect according to the corresponding one of the exemplary embodiments described above, in any one of the first to seventh aspects, it is preferable that electrolytic liquid (W2) is ozone water.

Electrolytic liquid generation system (1, 1A, 1B, 1C) described above can generate ozone water (W2) effective for disinfection, deodorization, organic substance decomposition, and the like.

Electrolytic liquid generation system (1, 1A, 1B, 1C) of a ninth aspect according to the corresponding one of the exemplary embodiments described above, in any one of the first to eighth aspects, preferably further includes power source (7) configured to apply voltage for performing electrolysis to functional unit (4).

Electrolytic liquid generation system (1, 1A, 1B, 1C) described above does not require to prepare a separate power source, and thus is improved in convenience.

Electrolytic liquid generation system (1, 1A, 1B, 1C) of a tenth aspect according to the corresponding one of the exemplary embodiments described above, in any one of the first to ninth aspects, preferably further includes liquid feeder (2) configured to feed liquid (W1) to liquid inflow path (31).

Electrolytic liquid generation system (1, 1A, 1B, 1C) described above does not require to prepare a separate liquid feeder such as a feedwater pump, and thus is improved in convenience.

Control system (10) of an eleventh aspect according to the corresponding one of the exemplary embodiments described above is a control system which is used for electrolytic liquid generation system (1, 1A, 1B, 1C) that performs the disinfection function of disinfecting liquid (W1, W3) while performing the electrolytic liquid generation function of generating electrolytic liquid (W2) from liquid (W1, W3) by electrolyzing liquid (W1, W3). Control system (10) includes controller (5) that controls at least one of the electrolytic liquid generation function or the disinfection function. Controller (5) operates in any of the multiple control modes including at least the first control mode and the second control mode. Controller (5) improves the electrolytic liquid generation function in the first control mode more than in the second control mode. Controller (5) improves the disinfection function in the second control mode more than in the first control mode.

Control system (10) described above can adjust a balance between disinfection of liquid (W1, W3) and generation efficiency of electrolytic liquid (W2).

### REFERENCE MARKS IN THE DRAWINGS

- 1, 1A, 1B, 1C: electrolytic liquid generation system
- 2: liquid feeder
- 31: liquid inflow path (liquid flow path)
- 4: functional unit
- 41: flow rate adjuster
- 42: electrolytic part
- 5: controller
- 7: power source
- 8: detector
- 81, 83: upstream detector (detector)
- 82, 84: downstream detector (detector)
- 9: operation input unit
- 10: control system
- W1: tap water (liquid)
- W2: ozone water (electrolytic liquid)
- W3: mixed liquid (liquid)

## Claims

1. An electrolytic liquid generation system comprising:
a liquid flow path that allows a liquid to flow through the liquid flow path;
a functional unit that comprises an electrolytic liquid generation function of generating an electrolytic liquid from the liquid by electrolyzing the liquid fed through the liquid flow path, and a disinfection function of disinfecting the liquid; and
a controller that controls at least one of the electrolytic liquid generation function or the disinfection function of the functional unit,
wherein
the controller is configured to operate in any one of a plurality of control modes including at least a first control mode and a second control mode,
the controller is configured to improve the electrolytic liquid generation function in the first control mode more than in the second control mode, and
the controller is configured to improve the disinfection function in the second control mode more than in the first control mode.

2. The electrolytic liquid generation system according to Claim 1, wherein
the functional unit comprises a flow rate adjuster that adjusts a liquid feed rate that is an amount of the liquid fed from the liquid flow path, and
the controller is configured to control the electrolytic liquid generation function by controlling the flow rate adjuster to decrease the liquid feed rate in the second control mode more than in the first control mode.

3. The electrolytic liquid generation system according to Claim 1 or 2, wherein
the functional unit comprises an electrolytic part that electrolyzes the liquid,
the controller is configured to control a current to be fed to the electrolytic part, and
the controller is configured to control the disinfection function by increasing the current in the second control mode more than the current in the first control mode.

4. The electrolytic liquid generation system according to Claim 2, wherein
the functional unit comprises an electrolytic part that electrolyzes the liquid,
the controller is configured to control a current to be fed to the electrolytic part, and
the controller is configured to control the disinfection function by decreasing the current in the second control mode less than the current in the first control mode.

5. The electrolytic liquid generation system according to any one of Claims 1 to 4, further comprising:
an operation input unit that receives a human operation,
wherein
the controller is configured to select any one of the plurality of control modes in accordance with the operation received by the operation input unit, and operate in the control mode which has been selected.

6. The electrolytic liquid generation system according to any one of Claims 1 to 4, further comprising:
a detector that detects a degree of contamination of at least one of the liquid or the electrolytic liquid,
wherein
the controller is configured to select any one of the plurality of control modes in accordance with the degree of contamination, and operate in the control mode which has been selected.

7. The electrolytic liquid generation system according to Claim 6, wherein
the controller is configured to operate in the first control mode when the degree of contamination is less than a mode threshold, and
the controller is configured to operate in the second control mode when the degree of contamination is equal to or more than the mode threshold.

8. The electrolytic liquid generation system according to any one of Claims 1 to 7, wherein
the electrolytic liquid is ozone water.

9. The electrolytic liquid generation system according to any one of Claims 1 to 8, further comprising:
a power source that applies voltage for performing the electrolysis to the functional unit.

10. The electrolytic liquid generation system according to any one of Claims 1 to 9, further comprising:
a liquid feeder that feeds the liquid to the liquid flow path.

11. A control system which is used for an electrolytic liquid generation system comprising an electrolytic liquid generation function of generating an electrolytic liquid from a liquid by electrolyzing the liquid and a disinfection function of disinfecting the liquid, the control system comprising:
a controller that controls at least one of the electrolytic liquid generation function or the disinfection function,
wherein
the controller is configured to operate in any one of a plurality of control modes including at least a first control mode and a second control mode,
the controller is configured to improve the electrolytic liquid generation function in the first control mode more than in the second control mode, and
the controller is configured to improve the disinfection function in the second control mode more than in the first control mode.
